(19) Europäisches Patentamt
European Patent Office
Office européen des brevets

(11) Veröffentlichungsnummer: **0 115 007**
**B1**

(12) **EUROPÄISCHE PATENTSCHRIFT**

(45) Veröffentlichungstag der Patentschrift: **26.11.86**

(21) Anmeldenummer: **83112722.0**

(22) Anmeldetag: **17.12.83**

(51) Int. Cl.⁴: **A 61 K 7/46**, C 07 C 59/125, C 07 C 59/60, C 07 C 51/367

(54) **Alkoxyessigsäuren, ein Verfahren zu ihrer Herstellung und ihre Verwendung.**

(30) Priorität: **29.12.82 DE 3248463**

(43) Veröffentlichungstag der Anmeldung: **08.08.84 Patentblatt 84/32**

(45) Bekanntmachung des Hinweises auf die Patenterteilung: **26.11.86 Patentblatt 86/48**

(84) Benannte Vertragsstaaten: **CH DE FR GB IT LI NL**

(56) Entgegenhaltungen:
**AT-B-330 364**
**CH-A-624 844**
**DE-C-642 344**
**DE-C-721 541**
**FR-A-897 289**
**US-A-1 920 137**

**CHEMICAL ABSTRACTS, Band 69, 1968, Columbus, Ohio, USA, KHAR'KOV et al. "Synthesis and cyclization of esters of 5-oxo-3-oxaheptanecarboxylic acids into homologs of dioxane and morpholinone", Seite 9045, Spalte 1, Abstract-Nr. 96 615c**
**PATENTS ABSTRACTS OF JAPAN, unexamined applications, Section C, Band 3, Nr. 114, 21. September 1979, THE PATENT OFFICE JAPANESE GOVERNMENT, Seite 45 C 59**

(73) Patentinhaber: **Haarmann & Reimer GmbH, Postfach 1253, D-3450 Holzminden (DE)**

(72) Erfinder: **Surburg, Horst, Dr., Sachsenring 10, D-3450 Holzminden (DE)**
Erfinder: **Hopp, Rudolf, Dr., Auf dem Gehrenkamp 28, D-3450 Holzminden (DE)**
Erfinder: **Müller, Hans- Otto, Dr.- Jasper- Strasse 32, D-3450 Holzminden (DE)**

(74) Vertreter: **Mann, Volker, Dr., c/o Bayer Aktiengesellschaft Zentralbereich Patente Marken und Lizenzen, D-5090 Leverkusen-Bayerwerk (DE)**

**EP 0 115 007 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft Alkoxyessigsäuren, ein Verfahren zu ihrer Herstellung und ihre Verwendung als Riechstoffe. Die Alkoxyessigsäuren können in Parfümkompositionen und parfümierten Produkten verwendet werden.

Costus-Wurzelöl wird wegen seiner animalischen Note in Parfümkompositionen verwendet. Als Duftstoff natürlichen Ursprungs hat es den Nachteil der schwierigen Herstellung aus den Naturprodukten und der nicht immer gleichbleibenden Qualität.

Es wurde gefunden, daß Alkoxyessigsäuren der Formel

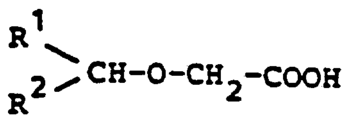

(I),

in der

$R^1$ Ethyl oder Vinyl und

$R^2$ Alkyl mit 3 bis 6 Kohlenstoffatomen bedeuten, eine ausgeprägte Costus-Note aufweisen und Costus-Wurzelöl ersetzen können. Die Erfindung betrifft daher die Verwendung von Alkoxyessigsäuren der Formel I als Riechstoffe.

Die 3-Hexyloxyessigsäure ist bekannt (Chem. Abstr. Vol. 69, 96615 c (1968)); sie wird als Nebenprodukt bei der Synthese von 5-Oxo-3-oxaheptancarbonsäuren erwähnt.

Im Rahmen der allgemeinen Formel I sind Alkoxyessigsäuren der Formel

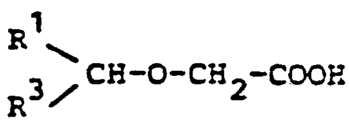

(II),

in der

$R^1$ Ethyl und

$R^3$ n-Alkyl mit 4 bis 6 Kohlenstoffatomen bedeutet,

neu.

Die Erfindung betrifft daher auch die neuen Alkoxyessigsäuren der Formel II.

Als Beispiele für die erfindungsgemäß zu verwendenden Alkoxyesigsäuren der Formel I seien genannt: 3-Hexyloxyessigsäure, 3-Heptyloxyessigsäure, 3-Octyloxyessigsäure, 3-Nonyloxyessigsäure und Oct-1-en-3-yloxyessigsäure.

Es wurde auch ein Verfahren zur Herstellung von Alkoxyessigsäuren der Formel II gefunden, das dadurch gekennzeichnet ist, daß man ein Alkoholat der Formel

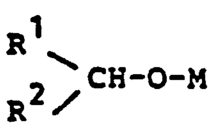

(III),

in der

$R^1$ und $R^3$ die unter Formel II angegebene Bedeutung haben und

M für Alkali steht,

mit einem Salz der Chloressigsäure umsetzt und das erhaltene Salz der Alkoxyessigsäure in an sich bekannter Weise in die freie Säure überführt.

Das erfindungsgemäße Verfahren kann anhand des folgenden Formelschemas erläutert werden:

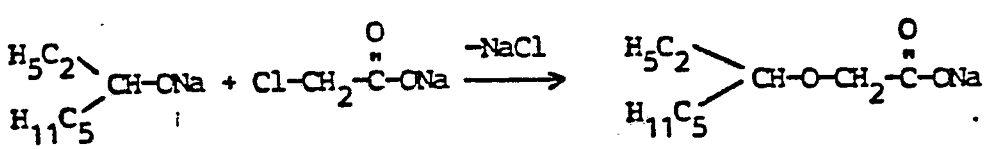

Alkoholate können erfindungsgemäß durch Umsetzung der entsprechenden Alkohole mit Alkalimetall oder einem Alkalihydrid hergestellt werden. Alkali kann hierbei erfindungsgemäß Lithium, Natrium, Kalium, Rubidium, oder Cäsium, bevorzugt Natrium oder Kalium, sein.

Die Überführung des Alkalisalzes der Alkoxyessigsäuren in die freie Säure kann beispielsweise durch die Zugabe einer Mineralsäure, wie Schwefelsäure, erfolgen. Im allgemeinen gibt man soviel an Mineralsäure zu, bis ein pH von etwa 1 erreicht ist.

Wenn man die Alkoholkomponente im Überschuß, beispielsweise von 4 bis 10 Mol, bevorzugt von 4 bis 6 Mol, bezogen auf 1 Mol des Natriumsalzes der Chloressigsäure, zugibt, kann das erfindungsgemäße Verfahren ohne Lösungsmittel durchgeführt werden.

Bei Durchführung des erfindungsgemäßen Verfahrens in Gegenwart eines inerten Lösungsmittels setzt man im allgemeinen etwa 1 bis 2 Mol, bevorzugt 1 bis 1,2 Mol, der Alkoholkomponente, bezogen auf 1 Mol des Natriumsalzes der Chloressigsäure, ein.

Inerte Lösungsmittel können beispielsweise aromatische Kohlenwasserstoffe wie Toluol, o-, m- und p-Xylol oder ein Gemisch der Xylolisomeren sein.

Bevorzugt dient als Lösungsmittel für das erfindungsgemäße Verfahren Toluol.

Das erfindungsgemäße Verfahren wird im allgemeinen im Temperaturbereich von 50 bis 150°C, bevorzugt von 100 bis 120°C, durchgeführt.

Das erfindungsgemäße Verfahren wird im allgemeinen bei Normaldruck durchgeführt. Es kann jedoch auch zweckmäßig sein, daß erfindungsgemäße Verfahren bei einem Unter- oder Überdruck durchzuführen. So kann beispielsweise das erfindungsgemäße Verfahren im Bereich von 0,2 bis 2 bar, bevorzugt von 0,3 bis 1,5 bar, durchgeführt werden.

Das erfindungsgemäße Verfahren wird beispielsweise wie folgt durchgeführt: In einem inerten Lösungsmittel stellt man durch Umsetzung des Alkohols mit Alkali oder Alkalihydrid das Alkalialkoholat her. Bei der Reaktionstemperatur gibt man dann unter Rühren das Natriumsalz der Chloressigsäure zu und kocht das Reaktionsgemisch unter Rückfluß. Bei Abkühlung fällt das Salz der Alkoxyessigsäure aus. Das Salz wird in Wasser gelöst und die Lösung mit Mineralsäure angesäuert. Aus der wäßrigen Lösung kann die freie Säure beispielsweise durch Extraktion abgetrennt werden.

Das natürliche Costus-Wurzelöl enthält eine Vielzahl von Komponenten wie Sesquiterpene und Sesquiterpen-Alkohole, Ketone und Lactone und weist einen sehr komplexen Geruch auf. Der Geruch kann als animalisch, bockig, ranzig, süßlich, Irisbutter-artig, wachsig-stearinig und fettalkoholisch (schmalzig) beschrieben werden. Überraschenderweise weisen die erfindungsgemäßen Alkoxyessigsäuren alle diese Merkmale des komplexen Costus-Geruchs auf und unterscheiden sich nur in der Betonung einzelner Noten.

Der Geruch der erfindungsgemäßen Riechstoffe läßt sich durch die folgende Geruchsbeschreibung wiedergeben:

3-Hexyloxyessigsäure:
feiner, guter Costus-Geruch mit Betonung der Fettalkohol-Note;

3-Heptyloxyessigsäure:
reintöniger Costus-Geruch, ausgewogenes Verhältnis von animalisch, ranzig, süßlich, Irisbutter-artig und bockig;

3-Octyloxyessigsäure:
gute Costusnote, schmalzig;

3-Nonyloxyessigsäure:
gute Costusnote, stark stearinig;

Oct-1-en-3-yl-oxyessigsäure:
gute Costusnote, fettig-ranzig.

Die erfindungsgemäßen Riechstoffe werden im allgemeinen in Lösungen eingesetzt. Als Lösungsmittel verwendet man im allgemeinen Phthalsäureester, Dipropylenglykol und Triethylcitrat.

Hervorgehoben sei eine 5 %-ige Lösung von 3-Heptyloxy-essigsäure in Triethylcitrat die in Stärke und Duftniveau dem natürlichen Costus-Wurzelöl entspricht.

Die erfindungsgemäßen Riechstoffe werden in Kombination mit anderen, an sich bekannten Riechstoffen (Arctander, Perfume and Flavor Chemicals, Montclair, N.J. (USA), 1969) und etherischen Ölen (Arctander, Perfume and Flavor Materials of Natural Origin., Elisabeth, N.J. (USA), 1960) angewendet und führen zu Parfümbasen und Riechstoffkompositionen mit ausdruckstarken Noten, die sich hervorragend für die Parfümierung von Fertigprodukten des Aerosol-, Waschmittel- und des Chemisch-Technischen-Sektors, insbesondere aber des Feinparfümerie- und Kosmetiksektors eignen, z.B. für Detergentien, Haarpflegemittel, Schaumbäder, Badesalz, Geschirrspülmittel, Waschpulver, Seifen, Antiperspirans, Puder, Cremes, Rasierwasser, After-Shave-Lotions, Raumluftverbesserer, WC-Reiniger, Raum-Sprays, Antiperspirans-Sprays, Deodorand-Sprays, Körper-Sprays, Insektizid-Sprays und Sonnenschutzmittel.

In diesen Präparaten werden die erfindungsgemäßen Riechstoffe im allgemeinen in einer Menge von 0,001 bis 1 Gew.-%, vorzugsweise von 0,001 bis 0,5 Gew.-%, bezogen auf das fertige Präparat, eingesetzt.

Die Herstellung der Parfümkompositionen und parfümierten Produkte erfolgt in üblicher Weise, beispielsweise durch Zusammengeben der Komponenten.

**Beispiel 1**

Zu 30 g Natriumhydrid in 400 g Toluol werden bei Siedetemperatur innerhalb von 30 Minuten 116 g 3-Heptanol zugetropft. Nach beendeter Zugabe wird 1 Stunde unter Rückfluß gekocht und auf 50°C abgekühlt. Dann werden unter kräftigem Rühren 117 g Natriumsalz der Chloressigsäure in mehreren Portionen innerhalb von 15 Minuten zugegeben und anschließend 16 Stunden unter Rückfluß gekocht. Das ausgefallene Salz wird in 400 g Wasser gelöst, die wäßrige Phase abgetrennt, mit 300 g 10-%iger Schwefelsäure angesäuert und mit 300 g Toluol extrahiert. Nach Entfernen des Lösungsmittels erhält man 120 g 3-Heptyloxyessigsäure, entsprechend 70 % der Theorie, mit einem Siedepunkt von 103°C bei 1,6 mbar.

**Beispiele 2-5**

Analog Beispiel 1 werden aus den in der folgenden Tabelle in Spalte 1 angeführten Alkoholen die in Spalte 2 angeführten Alkoxyessigsäuren mit den in Spalte 3 angegebenen Siedepunkten erhalten.

| Beispiel | 1 | 2 | 3 |
|---|---|---|---|
| 2 | 3-Hexanol | 3-Hexyloxyessigsäure | 86°C/0,9 mbar |
| 3 | 3-Octanol | 3-Octyloxyessigsäure | 106°C/0,7 mbar |
| 4 | 3-Nonanol | 3-Nonyloxyessigsäure | 120°C/1,5 mbar |
| 5 | Oct-1-en-3-ol | Oct-1-en-3-yloxyessigsäure | 103°C/0,7 mbar |

**Beispiel 6**

Eine Riechstoffkomposition vom Chypre-Typ wird durch Mischen folgender Komponenten hergestellt:

| | |
|---|---|
| 350 | Citronenöl |
| 30 | Citral |
| 1 | Tridecenal |
| 354 | Bergamottöl Reggio |
| 80 | Lavendelöl |
| 5 | Cumarin |
| 10 | Basilikumöl |
| 25 | Bayöl |
| 10 | Allyljonon |
| 10 | Jasmin absolute marokkanisch |
| 60 | Methyldihydrojasmonat |
| 20 | Vetiverylacetat |
| 20 | Patschuliöl |
| 20 | Sandelholzöl ostindisch |
| 995 | Gewichtsteile |

Durch den Zusatz von 5 Gew.-Teilen einer 5 %-igen Lösung von 3-Heptyloxyessigsäure in Triethylcitrat wird der Duftkomplex positiv abgerundet. Die vorher eher herbmaskuline Duftnote erhält durch den Zusatz eine feminine Betonung.

**Beispiel 7**

Ein "Femme-Akkord" wird durch Mischen der folgenden Komponenten hergestellt:

| | |
|---|---|
| 50 | Undecalacton |
| 30 | Macisöl |
| 10 | Cardamomenöl |
| 5 | Vanillin |
| 300 | Methyljonon (Isomerengemisch) |
| 300 | Patschuliöl |
| 300 | Abs. Mousse de Chêne jugosl. 50 % in Triethylcitrat |
| 995 | Gewichtsteile |

Der Zusatz von 5 Gew.-Teilen einer 5 %igen Lösung von 3-Heptyloxyessigsäure verleiht der Riechstoffkompositon eine komplettierende animalisch-würzige Abrundung.

**Patentansprüche**

1. Verwendung von Alkoxyessigsäuren der Formel

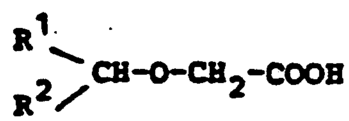

,

in der
$R^1$ Ethyl oder Vinyl und
$R^2$ Alkyl mit 3 bis 6 Kohlenstoffatomen bedeutet
als Riechstoffe.

2. Parfümkompositionen und parfümierte Produkte, enthaltend eine Komponente bestehend aus Alkoxyessigsäuren der Formel

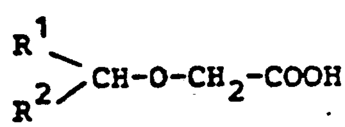

,

in der
$R^1$ Ethyl oder Vinyl und
$R^2$ Alkyl mit 3 bis 6 Kohlenstoffatomen bedeutet.

3. Parfümkompositionen und parfümierte Produkte nach Anspruch 2, enthaltend 0,001 bis 1 Gew.-% der Alkoxyessigsäure bezogen auf das fertige Präparat.

4. Neue Alkoxyessigsäuren der Formel

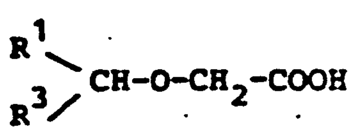

,

in der
$R^1$ Ethyl und
$R^3$ n-Alkyl mit 4 bis 6 Kohlenstoffatomen bedeutet.

5. Verfahren zur Herstellung der Alkoxyessigsäuren, gemäß Anspruch 4, dadurch gekennzeichnet, daß man ein Alkoholat der Formel

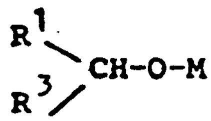,

in der
$R^1$ Ethyl
$R^3$ n-Alkyl mit 4 bis 6 Kohlenstoffatomen bedeutet, und
M für Alkali steht,
mit einem Alkalisalz der Chloressigsäure umsetzt und das erhaltene Alkalisalz der Alkoxyessigsäure in an sich bekannter Weise in die freie Säure überführt.

**Claims**

1. Use of alkoxyacetic acids of the formula

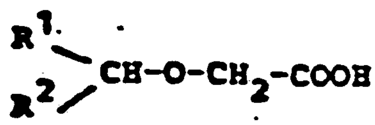,

in which
$R^1$ denotes ethyl or vinyl and
$R^2$ denotes alkyl with 3 to 6 carbon atoms,
as odoriferous substances.

2. Perfume compositions and perfumed products containing a component consisting of alkoxyacetic acids of the formula

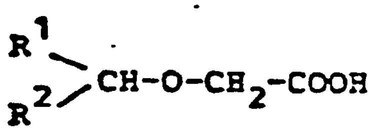,

in which
$R^1$ denotes ethyl or vinyl and
$R^2$ denotes alkyl with 3 to 6 carbon atoms.

3. Perfume compositions and perfumed products according to Claim 2, containing 0.001 to 1% by weight of the alkoxyacetic acid, relative to the finished preparation.

4. New alkoxyacetic acids of the formula

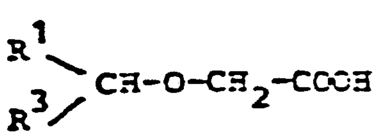,

in which
$R^1$ denotes ethyl and
$R^3$ denotes n-alkyl with 4 to 6 carbon atoms.

>5. Process for the preparation of the alkoxyacetic acids according to Claim 4, characterised in that an alcoholate of the formula

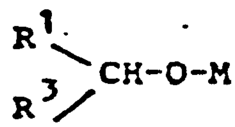,

in which
$R^1$ denotes ethyl,
$R^3$ denotes n-alkyl with 4 to 6 carbon atoms and
M represents alkali metal,
is reacted with an alkali metal salt of chloroacetic acid and the alkali metal salt of the alkoxyacetic acid obtained is converted into the free acid in a manner known per se.

## Revendications

1. Utilisation d'acides alkoxyacétiques de formule

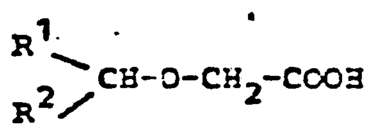,

dans laquelle
$R^1$ est un groupe éthyle ou vinyle et
$R^2$ est un groupe alkyle ayant 3 à 6 atomes de carbone,
comme produits odorants.

2. Compositions de parfums et produits parfumés, contenant un composant constitué par des acides alkoxyacétiques de formule

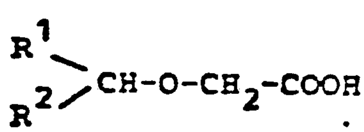,

dans laquelle
$R^1$ est un groupe éthyle ou vinyle et
$R^2$ est un groupe alkyle ayant 3 à 6 atomes de carbone.

3. Compositions de parfums et produits parfumés suivant la revendication 2, contenant 0,001 à 1% en poids de l'acide alkoxyacétique par rapport à la préparation prête à l'emploi.

4. Acides alkoxyacétiques nouveaux de formule

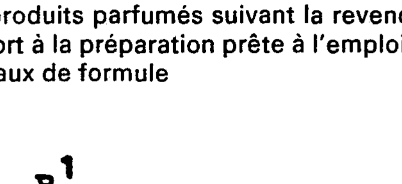,

dans laquelle
$R^1$ est le groupe éthyle et
$R^3$ est un groupe n-alkyle ayant 4 à 6 atomes de carbone.

5. Procédé de production des acides alkoxyacétiques suivant la revendication 4, caractérisé en ce qu'on fait réagir un alcoolate de formule

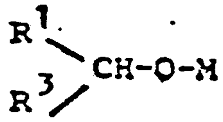

dans laquelle
$R^1$ est le groupe éthyle,
$R^3$ est un groupe n-alkyle ayant 4 à 6 atomes de carbone, et
M est un métal alcalin,
avec un sel de métal alcalin de l'acide chloracétique et on transforme en l'acide libre, d'une manière connue le sel de métal alcalin de l'acide alkoxyacétique que l'on obtient.